# FASCICULE DE BREVET EUROPEEN

(11) **EP 1 051 617 B1**
(45) Date de publication et mention de la délivrance du brevet: **07.04.2004**
(21) Numéro de dépôt: 98958987.4
(22) Date de dépôt: 08.12.1998
(51) Int. Cl.: G01N 33/487

(54) **DISPOSITIF JETABLE POUR LE PRELEVEMENT D'UN LIQUIDE BIOLOGIQUE OU MINERAL AVEC DETECTION ET/OU DOSAGE D'UN PARAMETRE BIEN DEFINI**
EINMALVORRICHTUNG ZUR AUFNAHME EINER BIOLOGISCHEN ODER MINERALISCHEN FLÜSSIGKEIT MIT ANALYSE MIT DETEKTION UND DOSIERUNG EINES DEFINIERTEN PARAMETERS
DISPOSABLE DEVICE FOR SAMPLING A BIOLOGICAL OR MINERAL LIQUID DETECTING AND/OR ASSAYING A SPECIFIC PARAMETER

(43) Date de publication de la demande: 15.11.2000
(73) Titulaire: Cohen, André, Dr., F-30000 Nimes (FR); Cohen, Hélène, 30000 Nimes (FR)
(72) Inventeur: Cohen, André, Dr., F-30000 Nimes (FR); Cohen, Hélène, 30000 Nimes (FR)
(86) Numéro de dépôt international: PCT/FR1998/002665
(87) Numéro de publication internationale: WO 2000/034775

(56) Documents cités:
- US-A- 3 640 388
- US-A- 5 024 238
- US-A- 5 301 685

## Description

L'invention est relative à un dispositif jetable, du type à aiguille et réservoir, pour le prélèvement de liquide biologique ou minéral, destiné à détecter et/ou à doser un paramètre biologique ou minéral bien défini, notamment le sucre dans le sang, au moyen d'un révélateur colorimètrique, chromogéne ou de tout autre type.

Les dispositifs connus destinés à prélever du liquide biologique ou minéral sont essentiellement des types suivants :
- aiguille-tube ou seringue à piston servant au prélèvement ou de chambre de prélèvement de liquide biologique par piqûre d'une veine ou d'une artère US 3,640,388 la détection ou le dosage d'un paramètre bien défini se faisant dans un deuxième temps au moyen d'un autre dispositif;
- auto-piqueur servant uniquement à faire couler une goutte de liquide biologique, la détection et le dosage d'un paramètre bien défini se faisant directement sur la goutte au moyen d'un révélateur approprié (bandelette colorimètrique par exemple).
Ces dispositifs nécessitent au moins deux opérations et ne sont pas adaptés à un usage fréquent, fiable et autonome comme par exemple celui du dépistage ou du contrôle du taux de sucre dans le sang chez les diabétiques plus particulièrement dans les pays sous-développés.

Le déposant a conçu et réalisé un dispositif jetable, autonome, utilisable par tous, de conception simple, prélevant quelques gouttes de liquide biologique dans une veinule sous-cutanée, à détection ou dosage en une seule opération, extemparanément, in situ.

Le dispositif, selon l'invention comporte un réservoir, qui est réalisé en matériau souple, et est du type apte à prélever, par relachement de la pression bidigitale exercée sur sa paroi, le volume nécessaire de liquide; le dispositif se caractérise en ce que le révélateur est placé à l'intérieur dudit réservoir et en ce que le prélèvement dudit liquide, la détection du paramètre recherché et/ou son dosage, se font extemporanément, en une seule opération, à l'intérieur dudit réservoir.

Selon diverses variantes de l'invention, le dispositif comporte:
- à l'entrée du réservoir, en vis à vis de l'aiguille, un filtre apte à retenir certains composants et à en laisser passer d'autres;
- dans le réservoir, un filtre qui sépare l'intérieur dudit réservoir en deux cavités qui peuvent contenir, pour la première, au moins un réactif et, pour la deuxième, un révélateur.

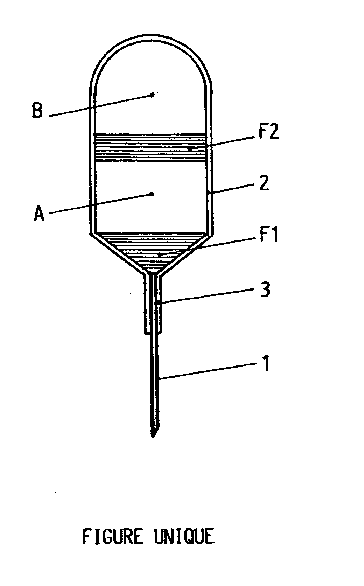

Les caractéristiques et les avantages de l'invention vont apparaître plus clairement à la lecture de la description détaillée qui suit d'au moins un mode de réalisation préféré de celle-ci donné à titre d'exemple non limitatif et représenté au dessin annexé (figure unique) qui est une vue en coupe longitudinale du dispositif selon l'invention.

Le dispositif représenté comporte une aiguille (1) et un réservoir (2), réalisé en matériau souple, apte à prélever, par relachement de la pression bidigitale exercée sur sa paroi, le volume nécessaire de liquide biologique ou minéral.

Le révélateur est placé à l'intérieur dudit réservoir et le prélèvement dudit liquide, la détection du paramètre recherché et/ou son dosage, se font, extemporanément, en une seule opération, in situ, à l'intérieur dudit réservoir.

Selon diverses variantes de l'invention, le dispositif comporte :
- à l'entrée du réservoir (2), en vis à vis de l'aiguille (1), un filtre (F1), fixe, apte à retenir certains composants et à en laisser passer d'autres;
- dans le réservoir (2), un réactif apte à réagir avec le ou les composants filtrés par le filtre (F1);
- dans le réservoir (2), un filtre (F2), fixe, qui sépare l'intérieur dudit réservoir en deux cavités (A) et (B) qui peuvent contenir, pour la première, au moins un réactif et, pour la deuxième, un révélateur.

Le filtre (F2) filtre, de la cavité (A) vers la cavité (B), le produit de la réaction du ou des composants filtrés par le filtre (F1) avec le ou les réactifs.

Le révélateur et les réactifs se présentent sous une forme choisie notamment parmi les formes suivantes : liquide, lyophilisée ou greffée aux éléments délimitant chaque cavité (filtres et parois intérieures du réservoir).

Dans le cas de la mesure du sucre dans le sang, le filtre (F1) retient les globules rouges et laisse passer le sérum ou le plasma, le réactif, choisi notamment parmi les enzymes, les co-enzymes et les tampons, est de la glucose-oxydase et le révélateur de la quinone-imine.

Une échelle graduée peut être associée au révélateur pour déterminer le dosage du paramètre recherché.

Dans la réalisation préférée de l'invention, l'aiguille utilisée est une aiguille du type aiguille d'acupuncture munie d'un canalicule et le réservoir associé est un micro-réservoir, en matériau souple, muni d'une embase (3) pour la fixation de ladite aiguille.

Ladite embase sert également de moyen de préhension de l'ensemble aiguille et réservoir.

Le réservoir peut prélever à chaque usage l'équivalent de 3 à 4 gouttes de liquide soit environ 0,1 millilitre.

Il peut être réalisé en polymère souple, translucide voire transparent, déformable (sans piston).

Le filtre (F1) est placé directement en sortie d'aiguille à l'entrée du réservoir (absence de chambre de prélèvement).

La séparation éventuelle de certains composants et la détection et/ou le dosage du paramètre recherché se font à l'intérieur du réservoir.

Le passage au travers du filtre (F2) se fait en plaçant la cavité (B) vers le bas.

Le réservoir est initialement vide de tout liquide.

Les aménagements à l'intérieur du corps du réservoir permettent :
- la mise en évidence du paramètre, qu'il soit sanguin, urinaire, humoral, d'origine humaine ou animale;
- le dosage du paramètre recherché;
et sont constitués par :
- l'apport de réactifs;
- des modifications structurelles du polymère du réservoir, de sa texture
ou de sa surface interne;
- l'apport de filtres (F1) et de filtres-membranes (F2) adaptés à la séparation des composants souhaités.

Dans le cas du dosage du sucre dans le sang :
- l'enzyme utilisée (gluco-oxydase) réagit avec le sérum pour donner de l'eau oxygénée;
- le révélateur utilisé (quinone-imine) donne un dérivé coloré (rose) dont l'intensité donnera le taux de glucose sanguin.

Les grosses molécules qui auraient pu réagir avec le révélateur ou gêner la réaction, sont stoppées par le filtre (F2).

Le réservoir ne peut comporter que le filtre (F1) et contenir les réactifs et le révélateur sous différentes formes et sur les différentes parties de la cavité.

Les filtres (F1) et (F2), déforniables et souples, sont fixés sur les parois internes du réservoir sans nécessiter de position particulière par rapport à l'axe de symètrie du dispositif.

Les filtres et les réactifs sont des types disponibles dans le commerce pour effectuer les opérations correspondantes.

Bien entendu, l'invention n'est pas limitée aux modes de réalisation décrits et représentés pour lesquels on pourra prévoir d'autres variantes, en particulier dans :
- les types et le nombre de réactifs et de révélateurs contenus dans le réservoir;
- les types et le nombre de filtres contenus dans ledit réservoir;
- la nature et la forme de la paroi du réservoir.

## Revendications

1. Dispositif pour le prélèvement de liquide biologique ou minéral dans le but d'y détecter et/ou d'y doser un paramètre biologique ou minéral, bien défini, au moyen de réactifs et de révélateurs colorimétriques, chromogènes ou de tout autres types;
le dispositif comportant une aiguille (1) et un réservoir (2) aptes à prélever, par relâchement de la pression bidigitale exercée sur sa paroi, le volume nécessaire de liquide biologique ou minéral **caractérisé en ce qu'**il comporte lesdits réactifs et révélateurs qui sont placés à l'intérieur dudit réservoir de manière à détecter et/ou à doser le paramètre recherché, extemporanément, en une seule opération, in situ, à l'intérieur dudit réservoir.

2. Dispositif, selon la revendication 1, **caractérisé en ce qu'**il comporte, à l'entrée du réservoir (2), en vis à vis de l'aiguille (1), un filtre (F1), fixe, apte à retenir certains composants et à en laisser passer d'autres.

3. Dispositif, selon la revendication 1 ou la revendication 2, **caractérisé en ce que** le réservoir (2) contient des réactifs et des révélateurs aptes à réagir avec le ou les composants prélevés.

4. Dispositif, selon l'une quelconque des revendications précédentes, **caractérisé en ce que** le réservoir (2) comporte un filtre (F2), fixe, qui sépare l'intérieur dudit réservoir en deux cavités (A) et (B).

5. Dispositif, selon les revendications 3 et 4, **caractérisé en ce que** les réactifs sont placés dans la cavité (A), délimitée par les filtres (F1) et (F2), et les révélateurs sont placés dans la cavité (B) située de l'autre côté du filtre (F2).

6. Dispositif, selon la revendication 5, **caractérisé en ce que** le filtre (F2), filtre, de la cavité (A) à la cavité (B), le produit de la réaction du ou des composants filtrés par le filtre (F1) avec les réactifs.

7. Dispositif, selon la revendication 5, **caractérisé en ce que** les réactifs et les révélateurs se présentent sous une forme choisie notamment parmi les formes suivantes: liquide, lyophilisée ou greffée aux éléments délimitant chaque cavité.

8. Dispositif, selon l'une quelconque des revendications 3, 5 et 7, **caractérisé en ce que**, dans le cas de la mesure du taux de sucre dans le sang, le filtre (F1) retient les globules rouges et laisse passer le sérum ou le plasma, le réactif, choisi notamment parmi les enzymes, co-enzymes et les tampons, est de la glucose oxydase et le révélateur de la quinone-imine.

9. Dispositif, selon l'une quelconque des revendications précédentes, **caractérisé en ce qu'**une échelle est associée aux révélateurs.

10. Dispositif, selon l'une quelconque des revendications précédentes, **caractérisé en ce que** l'aiguille utilisée est une aiguille du type aiguille d'acupuncture munie d'un canalicule et le réservoir associé est un micro-réservoir, en matériau souple, muni d'une embase (3) pour la fixation de ladite aiguille.

## Patentansprüche

1. Vorrichtung für die biologische oder mineralische Flüssigkeitsabgabe
mit dem Ziel, dort festzustellen und/oder dort einen gut definierten biologischen oder mineralischen Parameter zu dosieren, mit Hilfe von reaktive Substanz und von Entwicklern, kolorimetrisch, chromogene oder von jeden anderen Typen;
die Vorrichtung, die eine Nadel (1) und einen Behälter (2) umfaßt ist geeignet zu entnehmen, durch Nachlassen des Bi-digitalen Druck, der auf seine Wand ausgeübt wurde, das notwendige Volumen von biologischer oder mineralischer Flüssigkeit,
charakterisiert in dem die besagte reaktive Substanze und Entwickler umfaßt sind,
und innerhalb des Behälters gesetzt, zur Festzustellung und/oder den gesuchten Parameter zu dosieren, unvorbereitet (aus dem Stehgreif),in nur einer Operation, in situ, innerhalb des Behälters.

2. Vorrichtung nach Forderung 1, charakterisiert in dem sie umfaßt,
am Eingang des Behälters (2) in gegenüber der Nadel (1), ein Filter (F1), befestigt und geeignet einige Bestandteile zurückzuhalten und davon andere übergehen zu lassen.

3. Vorrichtung nach Forderung 1 oder Forderung 2, charakterisiert, in dem der Behälter (2) reaktive Substanze und Entwickler enthält, geeignet ist mit den entnommenen Bestandteilen zu reagieren.

4. Vorrichtung nach irgendeiner einer der vorhergehenden Forderungen,
charakterisiert in dem der Behälter (2) ein fester Filter (F2) umfaßt, wer trennt das Innere des Behälters in zwei Hohlräumen (A) und (B).

5. Vorrichtung nach Forderung 3 und 4, charakterisiert in dem die reaktive Substanze in den Hohlraum (A) gesetzt werden, abgegrenzt durch die Filter (F1) und (F2),
und die Entwickler werden in den Hohlraum (B) angesiedelt an einer anderen Seite des Filters (F2).

6. Vorrichtung nach Forderung 5, charakterisiert in dem der Filter (F2) filtriert, vom
Hohlraum (A) zum Hohlraum (B), das Produkt der Reaktion der durch den Filter (F1) filtrierten Bestandteile mit der reaktive Substanz.

7. Vorrichtung nach Forderung 5, charakterisiert in dem die reaktive Substanz und die Entwickler sich in einer ausgewählten Form stellen, insbesondere unter den folgenden
Formen: Flüssig, lyophilisiert oder veredelt an den Elementen, die jeden Hohlraum abgrenzen.

8. Vorrichtung nach irgendeiner einer Forderung 3,5 und 7, charakterisiert in dem
im Falle der Maßnahme des Zuckersatzes im Blut, der Filter (F1) die roten Körperchen zurück hält und das Serum oder das Plasma übergehen läßt, die reaktive Substanze sind insbesondere ausgewählt unter den Enzymen, Co Enzymen und die Pfropfen (Puffer), sie sind aus Glucose Oxydase, und der Entwickler ist Quinone Imine

9. Vorrichtung nach irgendeiner einer der vorhergehenden Forderungen, charakterisiert in dem Mann ein Maßstab mit den Entwickler verbunden kann.

10. Vorrichtungen nach irgendeiner einer der vorhergehenden Forderungen,
die sich charakterisiert in dem die benutzte Nadel eine Nadel des Typs Akupunktumadel ist, durchbohrt durch einen kleinen Kanal und, dass der assoziierte Behälter ein Mikrobehälter aus flexiblem Material ist, ausgestattet mit einem Unterteil (3) für die Befestigung der Nadel.

## Claims

1. Device for taking sample of biological or mineral liquid with the aim to detect or/and to measure in, a biological or mineral parameter, well defined, by the means of reagent and colourimetric tracers, chromogene and of all other type.
The device comprising a needle and a tank suited to take sample, by loosing the bi digital pressure exerted on his wall, the necessary volume of biological or mineral liquid,
**characterised in that** it comprises the known as reagent and tracer, which are set inside the known as tank by way to detect and/or to measure the required parameter, extemporanement, in a sole operation, in situ, inside the known as tank.

2. Device, according the claim 1, **characterised in that** it comprises, at the tank mouth (2), in facing needle (1), a filter (F2), fixed, suited to keep some components and to let some pass of others to pass.

3. Device, according the claim 1 or the claim 2, **characterised in that** the tank (2) contains reagent and tracer suitable to react with the taken components.

4. Device, according any number of previous claim, **characterised in that** the tank (2) contains a filter, (F2) fixed, dividing inside the known as tank in two cavities (A) and (B).

5. Device, according the claim 3 and 4, **characterised in that** the reagents are set in the cavity (A), demarcated by filters (F1) and (F2) and the tracers are set in the cavity (B) located by the other side niter (F2).

6. Device, according the claim 5, **characterised in that** the filter (F2), filters, from the cavity. (A) to the cavity (B), the reaction product of components filtered through the filter (F1) with reagents.

7. Device, according the claim 5, **characterised in that** reagents and tracers are presented in a form selected among those following forms: liquid, freeze-dried or graft onto elements demarcating each cavity.

8. Device, according any number claims 3, 5 et 7, **characterised in that**, in the case of measuring glycaemia, the filter keeps red cells and let pass serum or plasma, the reagent selected among enzyme, co-enzyme and buffer is oxidase glucose and the tracer is quinone imine.

9. Device, according any number of previous claims, **characterised in that** the a ladder is joined with tracers.

10. Device, according any number of previous claims, **characterised in that** the used needle is from a needle acupuncture type provided with a canalicus and the associated tank is in a flexible material provided with a base plate for fixing the known as needle.
